# EUROPEAN PATENT SPECIFICATION

(11) **EP 3 300 681 B1**
(45) Date of publication and mention of the grant of the patent: **06.11.2019**
(21) Application number: 17201985.3
(22) Date of filing: 16.04.2008
(51) Int. Cl.: A61B 18/14, A61B 90/00, A61B 17/00, A61B 18/00

(54) **ABLATION CATHETER WITH FLEXIBLE TIP**
ABLATIONSKATHETER MIT FLEXIBLER SPITZE
CATHÉTER D'ABLATION AVEC POINTE SOUPLE

(30) Priority: 23.05.2007 US 939799 P; 11.09.2007 US 853759
(43) Date of publication of application: 04.04.2018
(62) Divisional of application: 15169906.3
(73) Proprietor: Irvine Biomedical, Inc., Irvine, CA 92614 (US)
(72) Inventor: Pappone, Carlo, 23870 Lecco (IT); De la Rama, Alan, Cerritos, CA California 90703 (US); Chen, Peter Cheng, Irvine, CA California 92603 (US); Hata, Cary, Irvine, CA California 92620 (US); Shimizu, Jared A., Carmichael, CA California 95608 (US)
(74) Representative: Kramer Barske Schmidtchen Patentanwälte PartG mbB

(56) References cited:
- US-A- 5 688 267
- US-A1- 2003 088 244
- US-A1- 2004 015 215
- US-A1- 2004 181 138
- US-A1- 2006 064 123
- US-A1- 2006 278 248

## Description

### BACKGROUND OF THE INVENTION

This invention relates generally to catheter devices, and more specifically to a flexible electrode structure for catheter tips.

Catheters are flexible, tubular devices that are widely used by physicians performing medical procedures to gain access into interior regions of the body. Catheters with one or more electrodes are generally known in the surgical art. Electrode catheters can be used for electrically mapping a body part, to deliver therapy to a body part, or both.

For treating heart conditions such as arrhythmia, catheters having ablation electrodes are used to create lesions in heart tissues. Such lesions may be linear lesions or single point lesions. A single point lesion, as its name implies, is created by applying ablation energy at a single point region of tissue. On the other hand, linear lesions involve applying ablation energy over a larger area in an elongated region of tissue. Linear lesions are known to have some advantages over mere single point lesions.

Creating a linear lesion with only a conventional tip electrode, however, is relatively time-consuming, labor-intensive, and generally impractical. A surgeon may attempt to use a typical single point electrode catheter to create a linear lesion, by carefully dragging the single point electrode tip across the tissue while applying energy to the tissue surface, but this is difficult to accomplish successfully,

U.S. Patent No. 5,487,385 discloses a flexible catheter having ring electrodes disposed along its flexible shaft for creating a linear lesion. A surgeon may lay the catheter shaft with the electrodes across a tissue area, and allow the consecutively-arranged ring electrodes to to ablate the target tissue using RF energy. The ring electrodes, however, must apply sufficient energy in order to create lesion areas that are connected, thus forming a single linear lesion. Applying too much RF energy, however, can cause unwanted tissue damage, and this arrangement often results in a series of spaced-apart single point lesions.

U.S. Patent No. 6,063,080 discloses a catheter having an elongated electrode. This electrode has micro-slotting or micro-apertures across its surface to improve flexibility of the electrode, thus allowing a surgeon to lay the elongated electrode across a tissue surface. Despite some desirable properties, such a longitudinal-type electrode has several disadvantages. For example, the electrode requires a spherical structure at its tip to prevent the electrode from penetrating tissue. Also, a longitudinal type electrode cannot effectively create a linear lesion when the electrode is laid across a tissue having ridges. Further medical devices are known from US 2004/015215 A1 and US 2006/278248 A1.

### BRIEF DESCRIPTION OF THE INVENTION

Advantageous embodiments of flexible electrodes for catheters, and catheter devices having such electrode are disclosed that provide, among other things, flexing and bending capability to the electrodes at the catheter tip to more effectively reach targeted tissues, even tissues having irregular surfaces with ridges and the like, and to more reliably create linear lesions on body tissue. The electrodes also are configured to provide a freedom of movement and shortening of a length of the catheter tip along its longitudinal axis to maintain surface contact with, for example, vibrating or moving tissue that is targeted for ablation.

According to the invention an ablation catheter as defined in claim 1 is provided. Preferred embodiments are defined in the dependent claims.

### BRIEF DESCRIPTION OF THE DRAWINGS

Figure 1 is a perspective view of an exemplary embodiment of a flexible tip electrode for a catheter.
Figures 1A-1C illustrate alternative embodiments of the flexible tip electrode shown in Figure 1.
Figure 2 is a perspective view of another embodiment of a flexible tip electrode for a catheter.
Figures 2A-2D illustrate alternative embodiments of the flexible tip electrode shown in Figure 2.
Figure 3 is a perspective view of another embodiment of a flexible tip electrode for a catheter.
Figure 3A is a side view of the tip electrode shown in Figure 3.
Figure 4 is a perspective view of another embodiment of a flexible tip electrode.
Figures 4A-4B are alternative embodiments of the flexible tip electrode shown in Figure 4.
Figure 5 is a perspective view of another embodiment of a flexible tip electrode for a catheter.
Figure 6 is a perspective view of another embodiment of a flexible tip electrode for a catheter.
Figures 7A-7C illustrate embodiments of catheters having flexible electrode tips in use.
Figures 8A-8C illustrate further embodiments of catheters having flexible electrode tips in use.
Figure 8D is a cross-sectional view of a portion of Figure 8B taken along line A-A.
Figure 8E schematically illustrates an altered cross sectional shape for the electrode tip shown in Figure 8C.
Figure 9 is a side elevational view of a portion of another embodiment of flexible tip electrode.
Figure 9A is a magnified view of a portion of Figure 9.
Figure 10 is a side elevational view of a section of the electrode tip shown in Figure 9.
Figure 11 is a view of an alternative section of an electrode tip.
Figure 12A is schematic illustration of a section of a tip electrode.
Figure 12B is a side elevational view of a flexible tip electrode showing a degree of flexing.
Figure 12C is a side elevational view of the tip electrode shown in Figure 12B being dragged across tissue having ridges thereon.
Figure 12D is aside elevational view of the tip electrode being dragged across smooth tissue surface.
Figure 13 is a side elevational view of a portion of the tip electrode shown in Figure 9.
Figure 13A is a side view of the tip electrode depicted in Figure 13 at rest.
Figure 13B is a side view of the tip electrode in Figure 13 when pressed against a tissue surface.
Figure 14A is a longitudinal cross-sectional view of a further embodiment of a tip electrode.
Figure 14B is a longitudinal cross-sectional view of still another embodiment of a tip electrode.
Figure 15A is an illustrative view showing an exemplary electrode gap for forming a flexible tip electrode.
Figure 15B is an illustrative view of another electrode gap for forming a flexible electrode tip.

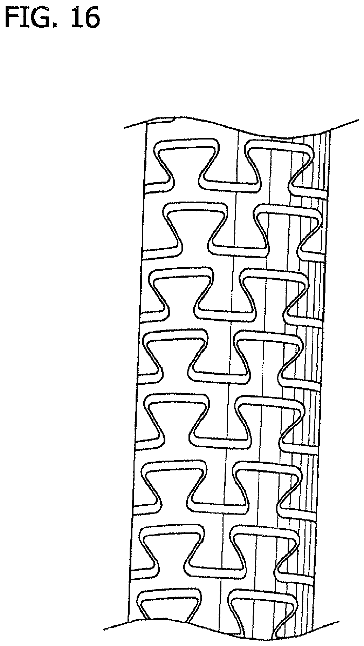

Figure 16 is a photograph of an exemplary tip electrode.

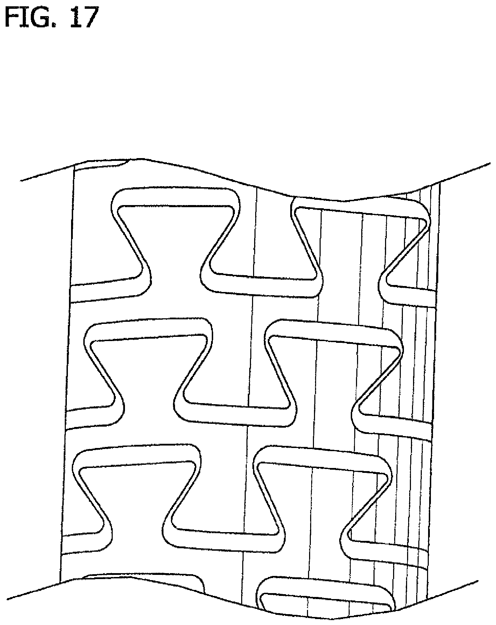

Figure 17 is a magnified view of a portion of the tip electrode shown in Figure 16.

### DETAILED DESCRIPTION OF THE INVENTION

Embodiments of ablation catheters having tips including flexible and bendable electrodes, and also freedom of movement to shorten an axial length of the catheter tip, while reliably creating linear lesions in body tissues are disclosed. The flexibility of the electrodes increases an electrode-to-tissue contact area, and in turn improves ablation of tissue. Especially in tissue where ridges are present, the flexible tip electrodes can be dragged across the ridges with improved continuous electrode-to-tissue contact.

These and other benefits are accomplished by providing a flexible tip electrode for an ablation catheter that includes a generally hollow cylindrical structure with an interior lumen. A round dome-shaped terminal end may be provided. The cylindrical wall of the electrode may have a variety of different types of elongated grooves or openings defining gaps in the cylindrical wall, imparting some flexibility to the electrode to more capably conform to and establish sufficient surface contact with body tissues that may have irregular surface area including ridges and the like, and tissues that may be vibrating or moving. The grooves, openings and associated elongated gaps may have various shapes, sizes and overall configurations as explained below in numerous exemplary embodiments.

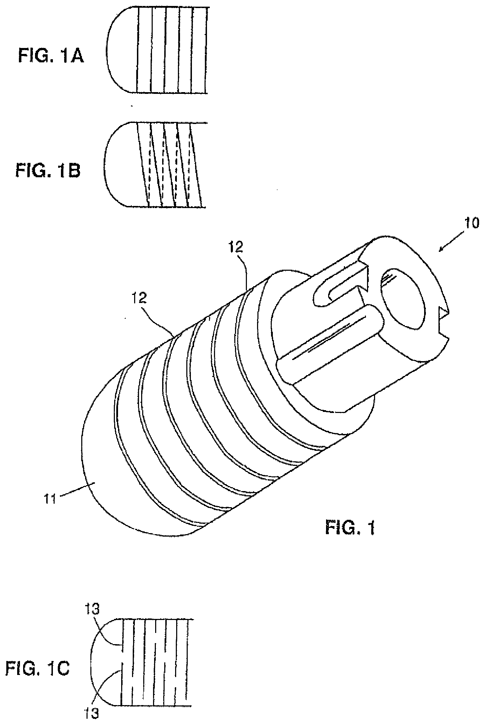

Referring now to Figure 1, an exemplary tip electrode 10 includes a hollow and generally cylindrical body having a dome tip 11 and a cylindrical sidewall. The sidewall may include a series of annular or ring-like surface channels or grooves 12 cut or otherwise formed into the sidewall. The grooves 12 define elongated areas of decreased wall thickness and decreased cross-sectional area of the sidewall, and hence the areas of the wall occupied by the elongated grooves 12 are structurally weaker and less rigid than areas of the sidewall where the grooves are not present, imparting flexible properties to the electrode wall. As used herein, an elongated groove preferably has a length that is at least about 3 times the width of the groove, more preferably at least about 5 times, and most preferably at least about 10 times.

As shown in Figure 1, the elongated grooves 12 are disposed about the tip electrode and extend generally parallel to one another. Each annular groove 12 extends in a plane that is generally perpendicular to a longitudinal axis of the tip. The respective grooves 12 may be spaced equidistant from each other along a longitudinal length of the tip electrode. Each annular groove 12 may form a continuous 360 degree unending loop (as illustrated in Figure 1A) that is circular. Alternatively, all or part of the series of ring grooves may extend in a non-circular and a non-planar helical configuration (as shown in Figure 1B) completing more than one 360 degree loop or turn on the surface of the electrode sidewall, with the helical ring grooves having discrete end points.

In another embodiment, the electrode may include some annular rings extending in a plane that do not form a continuous unending loop, but rather grooves forming loops having two terminal ends 13 (as depicted in Figure 1C) that are spaced apart from one another. A further embodiment may include a combination of continuous and non-continuous, planar and non-planar groove configurations.

In alternative embodiments, elongated openings extending completely through the thickness of the sidewall of the electrode may be provided in lieu of elongated surface channels or grooves. As used herein, an elongated opening preferably has a length that is at least about 3 times the width of the opening, more preferably at least about 5 times, and most preferably at least about 10 times.

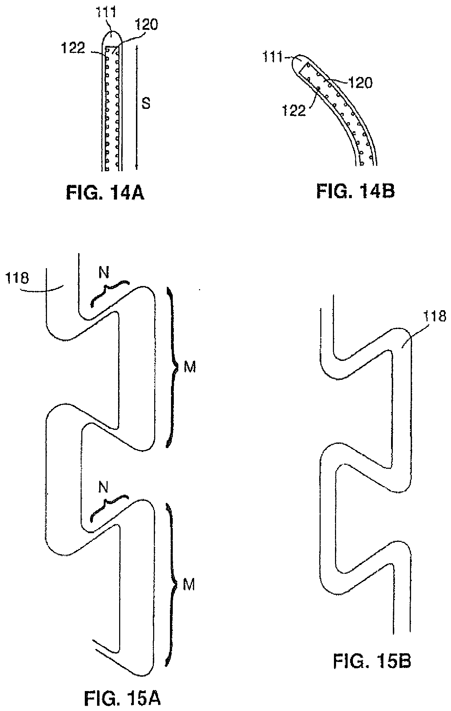

Elongated openings extending completely through the electrode sidewall will generally impart more flexibility, or less rigidity, in the sidewall than will elongated surface channel grooves. In the embodiment shown in Figure 1A, however, if each complete loop is completely cut through the sidewall, some type of additional supporting structure is required to connect the severed-pieces together. For example, a biasing element such as an inner coil may be provided within the lumen, such as in the embodiments of Figures 14A and 14B to be described further below.

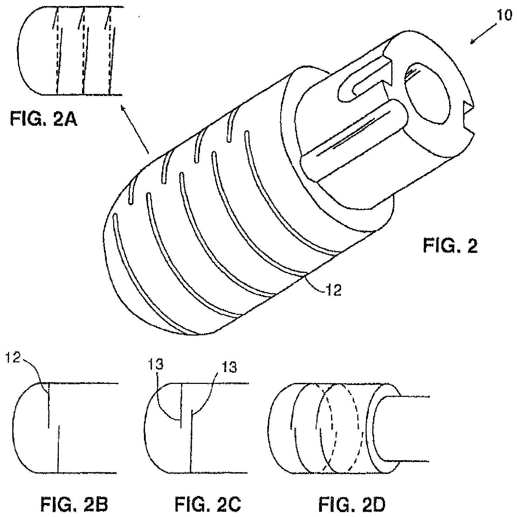

Referring now to Figure 2, the elongated ring-like, annular grooves 12 may be spaced further apart than in the embodiment shown in Figure 1. Also, in the embodiment of Figure 2, each ring-like, annular groove does not form a continuous, 360 degree loop on the electrode sidewall, and terminal ends of each respective groove are slightly offset or staggered relative to one another to maintain some degree of desired rigidity in the electrode sidewall. As shown in Figures 2A through 2C, the elongated grooves may have a circumferential length chosen so that the terminal ends 13 of adjacent grooves extend past one another for a specified distance in an interleaved or dovetail arrangement on the electrode sidewall. It is contemplated that in other embodiments a combination of continuous ring-like grooves, such as those shown in Figure 1, and non-continuous grooves such as those shown in Figure 2 may be utilized.

Figure 2D illustrates another embodiment where offset elongated ring-like grooves 12 extend in respective planes spaced along a longitudinal axis of the tip, but the ring-like grooves extend only partly around the cylindrical sidewall of the electrode. In the example shown in Figure 2D, the elongated grooves 12 extend as staggered half loops extending across about 180 degrees of the cylindrical circumference of the electrode wall. Many other relative positions of half loops are also contemplated. In other embodiments, some or all the elongated grooves 12 may extend across more or less than 180 degrees of the cylindrical circumference of the electrode sidewall.

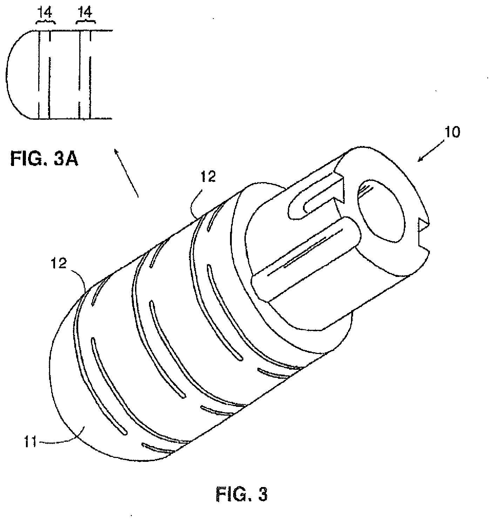

Figure 3 illustrates a tip electrode wherein three sets of elongated grooves 12 are provided, with each set 14 including two non-continuous ring-like loops. Spacing between the sets 14 is generally greater than spacing between the two ring-like loops in each set.

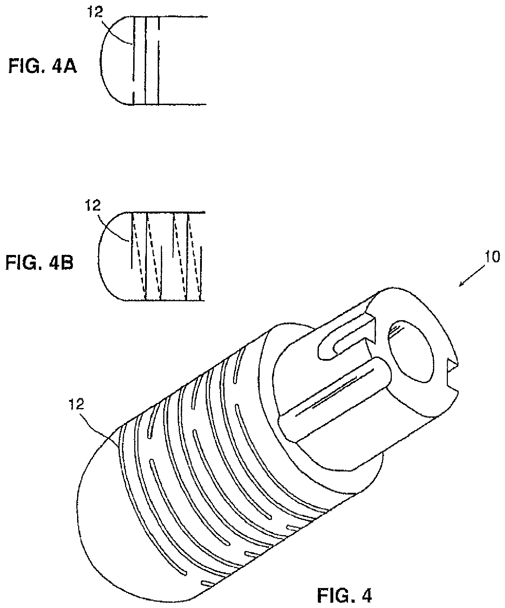

Figure 4 illustrates still further embodiments of a tip electrode. In the embodiment shown in Figure 4A, the elongated ring-like grooves form non-continuous loops on the outer surface of the electrode sidewall, with each groove having terminal ends that do not connect with each other. In the embodiment illustrated in Figure 4B, a series of elongated helical grooves 12 are provided that each extend for a number of turns on the surface of the electrode wall.

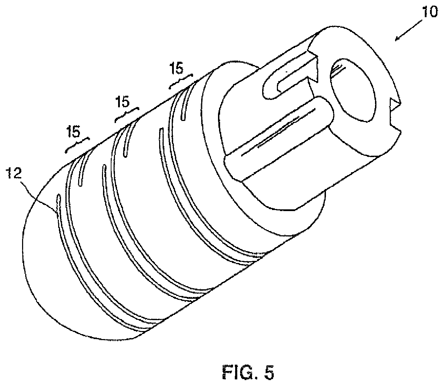

Figure 5 illustrates another catheter tip including three sets of elongated grooves 12. Each set 15 is shown to include a helical groove extending more than one 360 revolution or turn on the electrode sidewall.

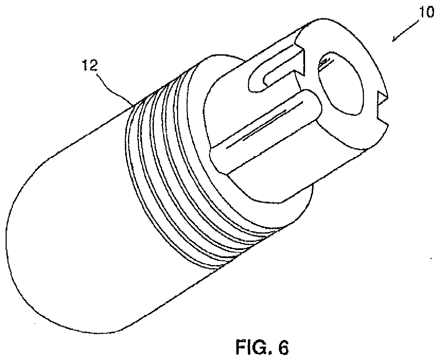

Figure 6 illustrates yet another contemplated embodiment where a series of elongated, ring-like grooves 12 are disposed generally equidistant from each other only along a proximal section of the tip electrode 10. Each ring-like groove 12 may or may not form a continuous loop or a complete turn on the electrode sidewall, A combination of continuous loops and non-continuous loops are possible. By providing the elongated grooves 12 in the proximal end of the electrode tip, but not the distal end, a desired rigidity in the distal portion of the tip electrode 10 may be provided, while affording some flexibility to the proximal portion of the tip.

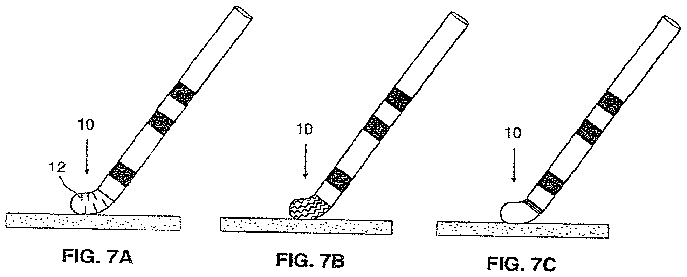

Figures 7A through 7C illustrate catheters including embodiments of flexible tip electrodes in use to map or ablate tissue surfaces. One aspect of the flexible tips allows and facilitates effective dragging of the flexible tip electrode 10 across a smooth tissue surface to create a linear lesion. This is possible because, as Figures 7A through 7C depict, the flexible tip electrode 10 may deform and/or flex when it is dragged across a tissue surface. The flexible and deformable properties of the flexible tips results in greater electrode-to-tissue surface area than would otherwise be possible with a rigid tip electrode.

In Figure 7A, the tip electrode includes an elongated cut pattern 12 that is similar to the embodiment depicted in Figure 2D including relatively parallel rings or loops, but extending completely through the thickness of the electrode wall to impart additional flexibility in comparison to the grooved embodiment of Figure 2D. In Figure 7B, a tip electrode with an elongated zig-zag cutting pattern allows even greater flexibility than the pattern of Figure 7A. In Figure 7C, however, the catheter tip electrode includes elongated grooves only at the neck region where the electrode is attached to the catheter (similar to the embodiment of Figure 6), allowing some degree of flexibility in a more rigid tip electrode.

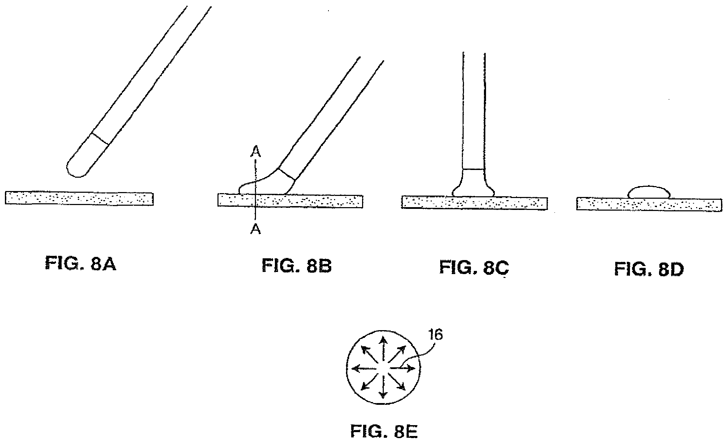

Figures 8A through 8E illustrate embodiments of the catheters having flexible tip electrodes that may advantageously deform, apart from simple bending along the longitudinal axis of the catheter body as shown in Figure 7, to create greater electrode-to-tissue surface area. In Figure 8A, a catheter including a flexible tip electrode is ready to make contact with a tissue surface. When the electrode makes contact with the tissue surface in Figure 8B, the tip electrode may deform. For example, a cross sectional area of the tip along line A-A in Figure 8B may become oval in shape as shown in Figure 8D. Such an electrode may not only flex along a longitudinal axis, but may also expand laterally when subjected to an applied force. When an angle between the tissue surface and a longitudinal axis of the catheter body gets closer to a 90 degree angle as shown in Figure 8C, the flexible tip may deform and shorten due to downward pressure against the tissue surface. Figure 8E, for example, schematically illustrates a cross section of the tip electrode in Figure 8C at a location parallel to the tissue surface. The electrode-to-tissue surface area, represented by the circle in Figure 8E is expandable outwardly in a direction of arrows 16 as the catheter is pressed further towards the tissue surface. One contemplated embodiment that has deformable properties as shown in Figures 8A through 8E is the tip electrode with an elongated zip-zag cut pattern as shown in Figure 7B.

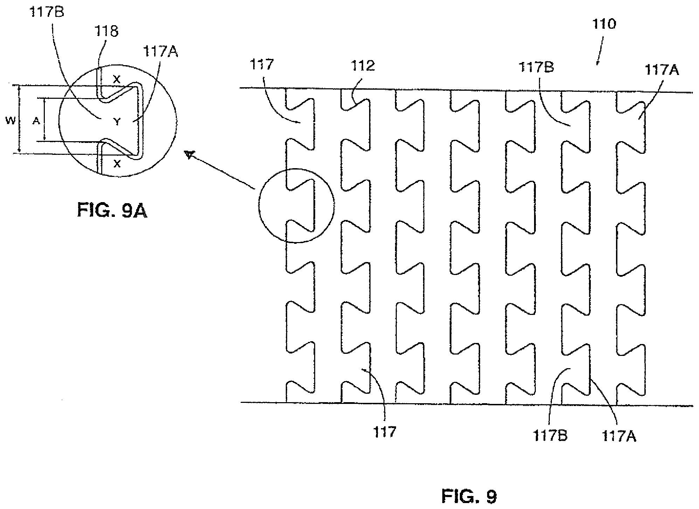

Referring now to Figure 9, an exemplary embodiment of flexible tip electrode 110 has an elongated cutting pattern in the electrode sidewall that outlines alternating interlocking blocks 117. In the illustrated embodiment, the contemplated blocks 117 are disposed on both sides of an elongated gap 118 created by the cutting pattern. Each block has a head 117A and a neck 117B, and the head 117A is wider than the neck 117B in each block. In the illustrated interlocking pattern, a first head, represented by "Y" in Figure 9A of the block 117, which has a neck 117B situated on one side of the gap 118, is disposed between a second and third heads represented by "X" in Figure 9A. The second and third heads X each have necks situated on the other side of the elongated gap 118 and opposing the head Y. The blocks X and Y are interlocked because the wider head portion of one block 117 is locked between the narrower neck portions of the two adjacent blocks 117. For example, the second and third heads X in Figure 9A are separated by a shortest distance A in Figure 9A, and distance A is shorter than a width W of the head Y, thereby restricting relative movement of two adjacent loops away from each other and preventing the blocks from separating.

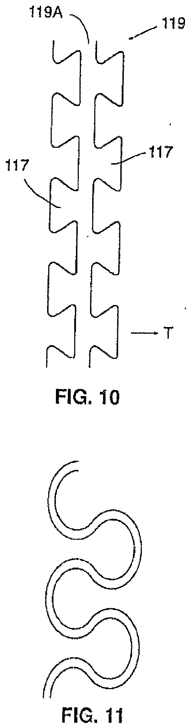

Contemplated patterns of elongated openings can also be described by focusing on the structures of the electrode wall, instead of focusing on the shape of the gap 118. For example, in Figure 10, a contemplated electrode wall includes a stem member 119 that may helically extend about a longitudinal axis of the electrode forming a series of stem loops (see Figure 9), and wherein the member 119 includes a plurality of protruding blocks 117 peripherally disposed on both sides of the stem member 119. Each block 117 transversely extends in a lateral direction indicated by arrow T in Figure 10 toward an adjacent stem loop in the electrode wall shown in Figure 9. Each adjacent stem loop includes blocks 117 that are staggered from the blocks 117 in immediately adjacent stem loops, resulting in an interlocking block pattern. Contemplated blocks for the stem member can have various shapes. For example, at least some of the blocks 117 may have a shape of an upside down triangle as illustrated, where one angle of the triangle represents the neck region. Alternatively, blocks with rounded bulbous shape such as ones shown in Figure 11 may alternatively be utilized. Contemplated heads of the bulbous shapes are wider than their corresponding necks, facilitating an interlocking block pattern.

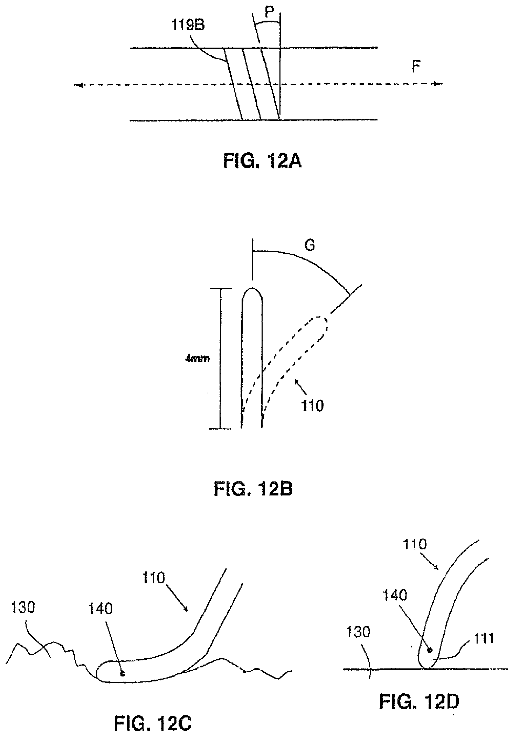

The stem members of Figures 10 and 11, for example, having an axis 119B, may extend in a helix about the longitudinal axis F in Figure 12A with a pitch P between and including 0.5 to 10 degrees. To describe it in another way, the patterns of elongated gaps 118 extend helically around the longitudinal axis F with a pitch angle, for example, between and including 0.5 to 10 degrees.

The contemplated elongated openings defining the gaps 118 between the blocks of the stem members (Figure 9) improve a flexibility of the electrode, and allow the electrode to flex and bend along the longitudinal length of the electrode and relative to the catheter body to which it is attached. For example, the ability of the electrode to flex allows an approximately 4 mm length of the electrode to bend at an angle G in Figure 12B that falls, for example, between and including 0.2 degrees to 70 degrees relative to the longitudinal axis from a substantially straight position. More specifically, the ability to flex allows the approximately 4 mm electrode length to bend between and including 5 degrees to 50 degrees relative to the longitudinal axis from a substantially straight position. Even more specifically, the ability to flex allows the approximately 4 mm length to bend about 45 degrees relative to the longitudinal axis from a substantially straight position.

Figures 12C and 12D illustrate a flexible electrode 110 being dragged across tissue 130. In Figure 12C, the electrode 110 is flexed and pressed against tissue 130, which has a relatively irregular surface. Being able to flex provides better contact with the target tissue, for example, in the trabeculated endocardial tissue where there are valleys, ridges, and pockets in the tissue surface. Here, electrode-to-tissue contact area is increased by using the side of the electrode 110 to deliver energy for ablation. The increased contact surface increases the likelihood of creating larger lesions at a given contact force and power setting. This in turn enables deeper ablation without having to increase the power setting, which is beneficial because increased power settings undesirably increase the likelihood of coagulation. In Figure 12D, the dome tip 111 is used to delivery energy to tissue 130.

The flexible electrode 110 also capably absorbs any contraction or vibration of tissue 130, and improves continuous tissue contact in a beating heart during systole and diastole, whether the electrode contacts the tissue 130 in a parallel, perpendicular, or other orientation, Continuous tissue contact is also assured regardless of whether the electrode is stationary at one location or when the electrode is in motion being dragged. Without such flexibility, a standard rigid tip electrode would "jump off" of the tissue in response to a beating heart.

According to the invention the electrode includes force-sensing capability to measure contact force with body tissue in different directions. For example, a strain gage, a fiber optic sensor, or other sensor 140 (Figure 12C) may be disposed within the hollow electrode to measure an amount of force causing the electrode to flex, and to shorten as the case may be. Such data can be collected and communicated to the physician to monitor ablation progress. Monitoring of force experienced at the electrode may, for example, be used to prevent accidental piercing of the target tissue via too much perpendicular force being applied to press the dome 111 into the tissue.

Unlike known elongated electrodes (e.g., U.S. Patent No. 6,063,080), which can be laid across a tissue to create relatively longer linear lesions, the flexible electrodes as described have the unexpected advantage of improving precision in mapping and control at specific locations within the heart for more precise ablation, especially in relatively tight anatomical structures. Known elongated electrodes have difficulty positioning in such tight anatomical structures.

One unexpected advantage achieved with a flexible tip electrode is minimized "flipping." When a standard rigid tip electrode is manipulated within a body cavity having valleys and pockets in the tissue, the tip electrode can get caught or stuck in the tissue. As a physician continues to apply force in an attempt to move the tip electrode even though it is caught or stuck, the tip electrode may suddenly "flip" out of the tissue. Such "flipping" is highly undesirable and should be avoided. The proposed flexible tip electrodes greatly minimize "flipping" issues, and allow smoother dragging and motion across valleys and pockets in target tissue.

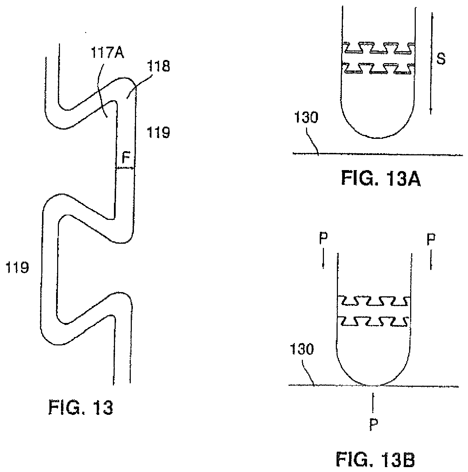

Referring now to Figure 13, the elongated openings in the wall provide a sufficient elongated gap 118 in the wall to allow shortening of a length of the electrode, when a force is applied to the electrode. The elongated gap 118 extends, for example, between a head 117A and a stem 119 of an adjacent loop in the electrode wall, and allows a freedom of movement F between adjacent stems along the longitudinal axis of the electrode wall when the elongated gap is narrowed or closed. Likewise, the elongated gap 118 between adjacent heads 117A provides a freedom of movement F for lengthening of the electrode along the longitudinal length of the electrode when the gap is opened or widened. Such shortening or lengthening may involve widening or narrowing one or more elongated gaps in the various embodiments described above.

In an exemplary embodiment, the electrode can shorten between and including 0.2% to 10% of an axial resting length of the electrode when the elongated gap(s) in the electrode wall are closed. In one embodiment the gap(s) in the electrode wall allows shortening of the axial length between and including 0.1% to 8% of the resting length. More specifically, the elongated gaps in the wall allow axial shortening of the length between and including 0.5% to 5% of the resting length, and even more specifically, the gaps in the wall allow shortening of the resting length between and including 0.1% to 0.5% of the length.

In Figure 13A, the electrode is at rest where no applied force is exerted thereon, and the electrode assumes a pre-determined shape stretching in the "S" direction and opening the elongated gap(s) to a predetermined amount. When the electrode contacts tissue 130 as shown in Figure 13B, an applied pressing force P causes the elongated gap(s) to narrow or close and the electrode to shorten, against the stretching force "S." Once shortened, the width of the elongated gap(s) providing freedom of movement F (Figure 13) in the direction of the applied force P may be minimized as depicted in Figure 13B. That is, the elongated gaps 118 may be fully closed such that the electrode length reaches a minimum axial length that is substantially unaffected by further exertion of applied force P.

In an exemplary embodiment, the stretching force "S" (Figure 13A) may be provided by a shape memory material or alloy used to fabricate the electrode wall. Alternatively, Figure 14A shows a cross sectional view of an electrode where the stretching force "S" is provided by a biasing element, such as a spring coil 122, in the lumen 120. The coil 122 provides structural integrity to the electrode wall and resiliently maintains the electrode in a pre-determined configuration in a resting state where no applied force is placed on the electrode. In one embodiment, the pre-determined electrode configuration at rest orients the longitudinal axis of the electrode to follow a straight line. In another embodiment, the pre-determined configuration at rest orients the longitudinal axis of the electrode along a curved or arcuate path as shown in Figure 14B. The contemplated coil resiliently biases the electrode to axially stretch in the direction of arrow S (Figure 14A) that is generally parallel to the longitudinal axis of the electrode. In other words, the coil optionally biases the tip electrode to stretch lengthwise. When deflected from the predetermined configuration under applied force, the electrode may resiliently return to the predetermined configuration when the applied force is released.

The coil 122, or the electrode, or both, may be fabricated from a shape memory material. The flexible tip electrode can be made of suitable conductive and biocompatible materials, suitable for ablation temperature; such materials include natural and synthetic polymers, various metals and metal alloys, Nitinol, naturally occurring materials, textile fibers, and combinations thereof. In one embodiment, the tip electrode is fabricated from MP3SN alloy.

Catheters having flexible tip electrodes such as those described above can optionally be coupled to an irrigation system. That is, the catheter may include a fluid delivery lumen in the tubular catheter body, with the fluid delivery lumen in fluid communication with the hollow electrode. When one or more of the flexible tip electrodes change shape under an applied force, the elongated gap(s) will undergo changes in size and/or shape, thereby affecting the fluid flow therethrough. A cooling fluid, for example, may be pumped in an open flow path through the catheter body to the hollow lumen of the electrode, where it may pass through the gap(s) in the electrode wall to the exterior of the electrode, bathing the electrode and adjacent body tissue with cooling fluid. An internal, closed-loop irrigation system using re-circulated cooling fluid as known in the art is also possible. Also, catheters having flexible tip electrodes can be coupled to an energy source, such as a radio frequency (RF) generator to provide energy needed for tissue ablation. RF signal generators are known and are disclosed, for example, in U.S. Patent No. 6,235,022.

Flexible tip electrodes for ablation catheters may be formed and fabricated, for example, according to the following methodology. An exemplary method includes providing a hollow cylindrical electrode, and applying a laser to the cylindrical wall of the electrode to cut through a wall of the electrode. The laser cuts the wall in a pre-determined pattern that may extend helically around the circumference of the electrode wall, or may conform to any of the elongated groove or opening patterns previously described in the various embodiments above. As shown in Figure 15, the cuts may create an elongated gap 118 that may be consistently wider in some sections M and narrower in some other sections N. The wider sections M may extend substantially laterally from or transverse to a longitudinal axis (such as the axis F in Figure 12) of the electrode wall. The narrower sections N may connect the wider sections M together, and may be disposed generally in the direction of the longitudinal axis F of the electrode wall.

The wider sections M allow freedom of movement to narrow or widen the gap(s) 118 as previously described, making it possible to shorten an axial length of the electrode when a force is applied at a distal end of the electrode towards a proximal end.

Figure 15B illustrates still another embodiment where the laser also cuts the electrode wall in a predetermined pattern, where the elongated gap 118 created by the laser has generally consistent width. A coil may be subsequently installed in the lumen of this electrode to provide stretching force to create wider sections and narrower sections as previously described in relation to Figure 14A.

Coatings such as gold and platinum can be applied to the electrode to increase thermo-conductivity of the electrodes. The electrodes can also be coated with heparin to provide anticoagulation effect. In addition, the electrodes may be electro-polished to reduce sharp edges.

The many embodiments of flexible electrodes facilitate the following exemplary methods of performing linear ablation procedures. As with typical ablation catheters, a physician can perform mapping using the electrodes, and determine a target site for ablation. Once determined, the physician may drag the flexible tip electrode across the target tissue to start ablation while applying energy to the tissue. Because the electrode is flexible, the electrode can be more easily dragged across tissue surfaces having ridges and bumps while keeping constant electrode-to-tissue contact. And because the gaps in the electrode wall allows the electrode to be shortened when pressed tip-down against tissue surface, accidental tissue-piercing is largely avoided if not eliminated.

Thus, specific embodiments and applications of flexible tip electrodes have been disclosed. It should be apparent, however, to those skilled in the art that many more modifications besides those already described are possible without departing from the inventive concepts herein. The inventive subject matter, therefore, is not to be restricted except by the scope of the appended claims.

## Claims

1. An ablation catheter comprising a catheter body and a hollow elongated tip electrode (10, 110) disposed at a distal end of the catheter body, the electrode (10, 110) configured to flex relative to a longitudinal axis of the catheter body, the electrode (10, 110) comprising a force-sensing member (140) configured to measure an amount of force contacting the electrode (10, 110), **characterised in that** the electrode (10, 110) further comprises a sidewall provided with one or more elongate gaps (12, 118) extending therethrough and providing a flexibility of the tip electrode (10, 110).

2. An ablation catheter in accordance with claim 1, wherein the force-sensing member (140) is configured to measure force in different directions.

3. An ablation catheter in accordance with any one of the preceding claims, wherein the force-sensing member (140) comprises one of a strain gage and a fiber optic sensor.

4. An ablation catheter in accordance with any one of the preceding claims, wherein the force-sensing member (140) is configured to measure the amount of force that causes at least one of flexing and shortening of the electrode (10, 110).

5. An ablation catheter in accordance with any one of the preceding claims, wherein the sidewall is a substantially cylindrical sidewall provided with at least one elongate gap (12) selected from the group consisting of an annular gap around a portion of a circumference of the sidewall and a helical gap forming a helical pattern on the sidewall.

6. An ablation catheter in accordance with any one of the preceding claims, wherein the sidewall is defined by a spiraling stem (119) extending about a longitudinal axis of the electrode (110), the stem (119) forming a series of turns and including a plurality of protruding blocks (117) extending toward an adjacent section of the stem (119), the gaps located between adjacent turns of the spiraling stem (119), and wherein the protruding blocks (117) are disposed on both sides of the stem (119), each protruding block (117) transversely extending towards an adjacent turn.

7. An ablation catheter in accordance with claim 6, wherein each protruding block (117) is configured to interlock with other protruding blocks (117).

8. An ablation catheter in accordance with any one of the preceding claims, wherein the electrode (10, 110) is configured to expand laterally.

9. An ablation catheter in accordance with any one of the preceding claims, wherein the electrode (10, 110) comprises a flexibility that enables it to radially deform such that a cross sectional shape of the electrode (10, 110) is changed.

10. An ablation catheter in accordance with any one of the preceding claims, wherein the electrode (10, 110) is configured to deform and flex when it is dragged across the tissue surface.

11. An ablation catheter in accordance with any one of the preceding claims, wherein the one or more elongate gaps (12, 118) are configured to allow a change of a length of the electrode (10, 110) under an applied force.

12. An ablation catheter in accordance with claim 4, wherein a configuration of the gaps (12, 118) is selected to shorten the electrode between about 0.1% to about 10% of a resting length of the electrode when the force is applied.

13. An ablation catheter in accordance with any one of the preceding claims, further comprising a biasing member (122) configured to impart a stretching force on the electrode (10, 110) and bias the electrode (10, 110) to stretch in an endwise direction relative to a longitudinal axis of the electrode, the biasing member (122) being provided within a lumen (120) of the ablation catheter.

14. An ablation catheter in accordance with any one of the preceding claims, further comprising a fluid delivery lumen in communication with the one or more elongate gaps (12, 118).

15. An ablation catheter in accordance with any one of the preceding claims, wherein the electrode (10, 110) is fabricated from a shape memory material configured to position the electrode in a pre-determined configuration.

## Patentansprüche

1. Ablationskatheter, der einen Katheterkörper und eine hohle längliche Spitzenelektrode (10, 110) aufweist, die an einem entfernten Ende des Katheterkörpers angeordnet ist, wobei die Elektrode (10, 110) zum Biegen relativ zu einer Längsachse des Katheterkörpers ausgebildet ist, wobei die Elektrode (10, 110) ein Kraft-sensierendes Element (140) aufweist, das zum Messen eines Betrags einer Kraft, die die Elektrode (10, 110) berührt, ausgebildet ist, **dadurch gekennzeichnet, dass** die Elektrode (10, 110) ferner eine Seitenwand aufweist, die mit einem oder mehreren länglichen Spalte (12, 118) versehen ist, die sich dort hindurch erstrecken und eine Flexibilität der Spitzenelektrode (10, 110) vorsehen.

2. Ablationskatheter nach Anspruch 1, wobei das Kraft-sensierende Element (140) zum Messen einer Kraft in verschiedenen Richtungen ausgebildet ist.

3. Ablationskatheter nach einem der vorhergehenden Ansprüche, wobei das Kraft-sensierende Element (140) eines aus einem Belastungsmessinstrument und einem Faseroptiksensor aufweist.

4. Ablationskatheter nach einem der vorhergehenden Ansprüche, wobei das Kraft-sensierende Element (140) zum Messen des Betrags einer Kraft ausgebildet ist, die wenigstens eines aus einem Biegen und Verkürzen der Elektrode (10, 110) bewirkt.

5. Ablationskatheter nach einem der vorhergehenden Ansprüche, wobei die Seitenwand eine im Wesentlichen zylindrische Seitenwand ist, die mit wenigstens einem länglichen Spalt (12) versehen ist, der aus der Gruppe ausgewählt ist, die aus einem ringförmigen Spalt um einen Bereich eines Umfangs der Seitenwand herum und einem spiralförmigen Spalt, der ein Spiralmuster auf der Seitenwand bildet, besteht.

6. Ablationskatheter nach einem der vorhergehenden Ansprüche, wobei die Seitenwand durch einen spiralartig ansteigenden Schaft (119) definiert ist, der sich um eine Längsachse der Elektrode (110) erstreckt, wobei der Schaft (119) eine Reihe von Windungen bildet und eine Vielzahl von vorstehenden Blöcken (117) aufweist, die sich in Richtung eines angrenzenden Abschnitts des Schafts (119) erstrecken, wobei sich die Spalte zwischen angrenzenden Windungen des spiralartig ansteigenden Schafts (119) befinden und wobei die vorstehenden Blöcke (117) auf beiden Seiten des Schafts (119) angeordnet sind, wobei jeder vorstehende Block (117) sich quer in Richtung zu einer angrenzenden Windung erstreckt.

7. Ablationskatheter nach Anspruch 6, wobei jeder vorstehende Block (117) zum Verriegeln mit weiteren vorstehenden Blöcken (117) ausgebildet ist.

8. Ablationskatheter nach einem der vorhergehenden Ansprüche, wobei die Elektrode (10, 110) ausgebildet ist, dass sie sich seitlich ausdehnt.

9. Ablationskatheter nach einem der vorhergehenden Ansprüche, wobei die Elektrode (10, 110) eine Flexibilität aufweist, die sie befähigt, sich radial derart zu verformen, dass sich eine Querschnittsform der Elektrode (10, 110) ändert.

10. Ablationskatheter nach einem der vorhergehenden Ansprüche, wobei die Elektrode (10, 110) zum Verformen und Biegen ausgebildet ist, wenn sie quer über die Gewebeoberfläche gezogen wird.

11. Ablationskatheter nach einem der vorhergehenden Ansprüche, wobei der eine oder die mehreren länglichen Spalte (12, 118) ausgebildet sind, dass sie eine Längenänderung der Elektrode (10, 110) unter eine aufgebrachten Kraft ermöglichen.

12. Ablationskatheter nach Anspruch 4, wobei eine Ausgestaltung der Spalte (12, 118) zum Verkürzen der Elektrode zwischen etwa 0,1% bis etwa 10% einer Länge der Elektrode im Ruhezustand, wenn die Kraft aufgebracht wird, ausgewählt ist.

13. Ablationskatheter nach einem der vorhergehenden Ansprüche, ferner enthaltend ein Vorspannelement (122), das zum Aufbringen einer Dehnungskraft auf die Elektrode (10, 110) und zum Vorspannen der Elektrode (10, 110) zum Dehnen in einer Längsrichtung relativ zu einer Längsachse der Elektrode ausgebildet ist, wobei das Vorspannelement (122) mit einem Lumen (120) des Ablationskatheters versehen ist.

14. Ablationskatheter nach einem der vorhergehenden Ansprüche, ferner enthaltend ein Fluidförderlumen in Verbindung mit dem einen oder mehreren länglichen Spalten (12, 118).

15. Ablationskatheter nach einem der vorhergehenden Ansprüche, wobei die Elektrode (10, 110) aus einem Formgedächtnismaterial hergestellt ist, das zum Positionieren der Elektrode in einer vorgegebenen Ausgestaltung ausgebildet ist.

## Revendications

1. Cathéter d'ablation comprenant un corps de cathéter et une électrode creuse à pointe allongée (10, 110) disposée à une extrémité distale du corps de cathéter, l'électrode (10, 110) étant configurée pour fléchir par rapport à un axe longitudinal du corps de cathéter, l'électrode (10, 110) comprenant un élément capteur de force (140) configuré pour mesurer une quantité de force en contact avec l'électrode (10, 110), **caractérisé en ce que** l'électrode (10, 110) comporte en outre une paroi latérale comportant un ou plusieurs espaces allongés (12, 118) s'y étendant au travers et offrant une flexibilité de l'électrode de pointe (10, 110).

2. Cathéter d'ablation selon la revendication 1, dans lequel l'élément capteur de force (140) est configuré pour mesurer la force dans différentes directions.

3. Cathéter d'ablation selon l'une quelconque des revendications précédentes, dans lequel l'élément capteur de force (140) comprend une jauge de contrainte et un capteur à fibre optique.

4. Cathéter d'ablation selon l'une quelconque des revendications précédentes, dans lequel l'élément capteur de force (140) est configuré pour mesurer la quantité de force qui provoque au moins une flexion et un raccourcissement de l'électrode (10, 110).

5. Cathéter d'ablation selon l'une quelconque des revendications précédentes, dans lequel la paroi latérale est une paroi latérale sensiblement cylindrique pourvue d'au moins un espace allongé (12) choisi dans le groupe constitué par un espace annulaire autour d'une partie d'une circonférence de la paroi latérale et un espace hélicoïdal formant un motif hélicoïdal sur la paroi latérale.

6. Cathéter d'ablation selon l'une quelconque des revendications précédentes, dans lequel la paroi latérale est définie par une tige en spirale (119) s'étendant autour d'un axe longitudinal de l'électrode (110), la tige (119) formant une série de tours et comprenant une pluralité de blocs saillants (117) s'étendant vers une section adjacente de la tige (119), les espaces situés entre des tours adjacents de la tige en spirale (119), et les blocs saillants (117) sont disposés des deux côtés de la tige (119), chaque bloc saillant (117) s'étendant transversalement vers un tour adjacent.

7. Cathéter d'ablation selon la revendication 6, dans lequel chaque bloc saillant (117) est configuré pour s'enclencher avec d'autres blocs saillants (117).

8. Cathéter d'ablation selon l'une quelconque des revendications précédentes, dans lequel l'électrode (10, 110) est configurée pour se dilater latéralement.

9. Cathéter d'ablation selon l'une quelconque des revendications précédentes, dans lequel l'électrode (10, 110) comprend une flexibilité qui lui permet de se déformer radialement de telle sorte qu'une forme en coupe transversale de l'électrode (10, 110) soit modifiée.

10. Cathéter d'ablation selon l'une quelconque des revendications précédentes, dans lequel l'électrode (10, 110) est configurée pour déformer et fléchir lorsqu'elle est traînée sur la surface du tissu.

11. Cathéter d'ablation selon l'une quelconque des revendications précédentes, dans lequel le ou les espaces allongés (12, 118) sont configurés pour permettre un changement de longueur de l'électrode (10, 110) sous une force appliquée.

12. Cathéter d'ablation selon la revendication 4, dans lequel une configuration des espaces (12, 118) est sélectionnée pour raccourcir l'électrode entre environ 0,1 % à environ 10 % de la longueur de repos de l'électrode lorsque la force est appliquée.

13. Cathéter d'ablation selon l'une quelconque des revendications précédentes, comprenant en outre un élément de sollicitation (122) configuré pour appliquer une force d'étirement sur l'électrode (10, 110) et solliciter l'électrode (10, 110) pour l'étirer dans une direction des extrémité par rapport à un axe longitudinal de l'électrode, l'élément de sollicitation (122) étant prévu dans une lumière (120) du cathéter d'ablation.

14. Cathéter d'ablation selon l'une quelconque des revendications précédentes, comprenant en outre une lumière d'administration de fluide en communication avec ledit un ou plusieurs espaces allongés (12, 118).

15. Cathéter d'ablation selon l'une quelconque des revendications précédentes, dans lequel l'électrode (10, 110) est fabriquée à partir d'un matériau à mémoire de forme configuré pour positionner l'électrode dans une configuration prédéterminée.
